(19) Europäisches Patentamt
European Patent Office
Office européen des brevets

(11) EP 0 610 407 B1

(12) EUROPEAN PATENT SPECIFICATION

(45) Date of publication and mention of the grant of the patent: **28.08.1996 Bulletin 1996/35**

(21) Application number: **92923542.2**

(22) Date of filing: **27.10.1992**

(51) Int. Cl.$^6$: **A61K 7/06**

(86) International application number: **PCT/US92/09237**

(87) International publication number: **WO 93/08787 (13.05.1993 Gazette 1993/12)**

# (54) SHAMPOO COMPOSITIONS WITH SILICONE, CATIONIC POLYMER, AND OILY LIQUID CONDITIONING AGENTS

HAARSHAMPOO-FORMULIERUNG MIT SILIKON, KATIONISCHEM POLYMER UND FLÜSSIGEN, ÖLIGEN KONDITIONIERUNGSMITTELN

COMPOSITIONS DE SHAMPOOING A BASE DE SILICIUM DE POLYMERE CATIONIQUE ET D'AGENTS DE MISE EN FORME HUILEUX LIQUIDES

(84) Designated Contracting States: **AT BE CH DE DK ES FR GB GR IE IT LI LU NL SE**

(30) Priority: **29.10.1991 US 784278**
**22.10.1992 US 960473**

(43) Date of publication of application: **17.08.1994 Bulletin 1994/33**

(73) Proprietor: **THE PROCTER & GAMBLE COMPANY**
**Cincinnati, Ohio 45202 (US)**

(72) Inventors:
- **WELLS, Robert Lee**
**Cincinnati, OH 45229 (US)**
- **SCHMIDT, Robert Raymond**
**Ft. Wright, KY 41011 (US)**
- **KING, Bonnie Theresa**
**Alexandria, KY 41001 (US)**

(74) Representative: **Brooks, Maxim Courtney**
**Procter & Gamble Limited**
**Whitley Road**
**Longbenton**
**Newcastle-upon-Tyne NE12 9TS (GB)**

(56) References cited:
**EP-A- 0 400 976**
**EP-A- 0 426 520**
**EP-A- 0 432 951**
**EP-A- 0 473 508**
**WO-A-92/10161**
**WO-A-92/10162**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Rank Xerox (UK) Business Services
2.13.3/3.4

## Description

This invention relates to shampoo compositions containing hair conditioning ingredients.

Human hair becomes soiled due to its contact with the surrounding atmosphere and, to a greater extent, from sebum secreted by the head. The build-up of the sebum causes the hair to have a dirty feel and an unattractive appearance. The soiling of the hair necessitates it being shampooed with frequent regularity.

Shampooing the hair cleans by removing excess soil and sebum. However, the shampooing process has disadvantages in that the hair is left in a wet, tangled and generally unmanageable state. Shampooing can also result in the hair becoming dry or "frizzy", and a loss of luster, due to removal of natural oils or other hair moisturizing materials. After shampooing, the hair can also suffer from a loss of "softness" perceived by the user upon drying. The hair can also suffer from increased levels of static upon drying after shampooing. This can interfere with combing and can result in fly-away hair. A variety of approaches have been developed to alleviate the after-shampoo problems. These range from the inclusion of hair conditioning aids in shampoos to post-shampoo application of hair conditioners, i.e., hair rinses. Hair rinses are generally liquid in nature and must be applied in a separate step following the shampooing, left on the hair for a length of time, and rinsed with fresh water. This, of course, is time consuming and is not as convenient as shampoos containing both cleaning and hair conditioning ingredients.

While a wide variety of shampoos have been disclosed which contain conditioning aids, they have not been totally satisfactory for a variety of reasons. Cationic conditioning agents are highly desirable for use in hair conditioning due to their abilities to control static, improve wet detangling, and provide a silky wet hair feel to the user. One problem which has been encountered in shampoos relates to compatibility problems between good cleaning anionic surfactants and the many conventional cationic agents which historically have been used as conditioning agents. Efforts have been made to minimize adverse interaction through the use of alternate surfactants and improved cationic conditioning agents. Cationic surfactants which provide good overall conditioning in hair rinse products, in general, tend to complex with anionic cleaning surfactants and provide poor conditioning in a shampoo context. In particular, the use of soluble cationic surfactants that form soluble ionic complexes do not deposit well on the hair. Soluble cationic surfactants that for: insoluble ionic complexes deposit on the hair but do not provide good hair conditioning benefits, and tend to cause the hair to have a dirty, coated feel. The use of insoluble cationic surfactants, e.g., tricetyl methyl ammonium chloride, can provide excellent anti-static benefits but do not otherwise provide good overall conditioning. Many cationic polymers tend to build up on the hair to result in an undesirable, "unclean" coated feel. Cationic polymers therefore, conventionally, are preferably used at limited levels to minimize this problem. This, however, can limit the overall conditioning benefits that are obtained. Additionally, cationic conditioning agents commonly do not provide optimal overall conditioning benefits, particularly in the area of "softness", especially when delivered as an ingredient in a shampoo composition.

Materials which can provide increased softness are nonionic silicones. Silicones in shampoo compositions have been disclosed in a number of different publications. Such publications include US-A-2,826,551, Geen, issued March 11, 1958; US-A-3,964,500, Drakoff, issued June 22, 1976; US-A-4,364,837, Pader, issued December 21, 1982; and GB-A-849,433, Woolston, issued September 28, 1960. While these patents disclose silicone containing compositions, they did not provide a totally satisfactory product in that it was difficult to maintain the silicone well dispersed and suspended in the product. Recently, stable, insoluble silicone-containing hair conditioning shampoo compositions have been described in US-A-4,741,855, Grote and Russell, issued May 3, 1988, US-A-4,788,066, Bolich and Williams, issued November 29, 1988, EP-A-0,400,976 and EP-A-0,426,520. These shampoo compositions can deliver excellent overall conditioning benefits to the hair while maintaining excellent cleaning performance, even with the use of anionic detersive surfactants, for a wide variety of hair types.

More recently, improved conditioning shampoos were provided in U.S. Serial No. 07/622,699*, Robert L. Wells, filed December 5, 1990, now abandoned, and its continuation application Serial No. 07/778,765*, filed October 21, 1991, wherein shampoos containing anionic surfactant, dispersed, insoluble silicone, and certain relatively low ionic strength cationic polymers were disclosed. These compositions provide excellent hair cleaning conditioning to a wide variety of hair types, especially including improved conditioning to hair damaged by color treatments, bleaching, permanents, etc.

JP-A-, Laid Open No. 56-72095, June 16, 1981, Hirota et al. (Kao Soap Corp.) also discloses shampoo containing cationic polymer and silicone conditioning agents. Still other patent publications relating to shampoos with cationic agents and silicone include EP-A-0 413 417, published February 20, 1991, Hartnett et al and EP-A-0,432,951

Another approach to providing hair conditioning benefits to shampoo compositions has been to use materials which are oily to the touch. These materials provide improved luster and shine to the hair. Oily materials have also been combined with cationic materials in the shampoo formulations. JP-A- Showa 53-35902, laid open October 6, 1979 (Showa 54-129135), N. Uchino (Lion Yushi Co.), discloses hair treatment compositions containing cationic polymer, fatty acid salt, and at (*equivalent to PCT/US91/08927, filed 29 November 1991) least 10% of an oily component for use before or after shampooing. Suitable oily components are hydrocarbons, higher alcohols, fatty acid esters, glycerides, and fatty acids. 62 [1987]-327266, filed December 25, 1987, published July 4, 1989, laid open No. HEI 1[1987]-168612, Horie et al., discloses detergent compositions containing cationic surfactant and/or cationic polymer, anionic surfactant,

and specific esters of the formula RCOOR' wherein R and R' are straight or branched chain alkyls.

In spite of these attempts to provide optimal combinations of cleaning ability and hair conditioning, it remains desirable to provide further improved hair conditioning shampoo compositions. For instance, it remains desirable to improve overall conditioning, and especially shine and luster, wet and dry combing, and dry hair feel, of hair treated with shampoo containing silicone and cationic material. For shampoos containing oily materials in combination with cationic materials, it remains desirable to improve overall conditioning, especially wet combing and detangling, dry combing, and dry hair feel. However merely increasing the level of one or both conditioning ingredients can result in adverse effects such as greasy hair feel and loss of fullness. it is desirable to improve conditioning without suffering from these drawbacks.

One attempt to do this is disclosed in EP-A-0 413 416, published February 20, 1991, Robbins et al., which discloses shampoo containing aminosilicone, anionic surfactant, cationic surfactant, and a hydrocarbon component. These types of formulations would normally be expected to result in either excessive buildup of aminosilicone on the hair, and consequently greasy hair feel and loss of fullness, or a relatively limited degree of improvement due to intentional use of very low levels of aminosilicone to avoid such adverse effects. The cationic surfactants would have limited ability to condition the hair due to interaction with the anionic surfactant.

EP-A-0 413 417, published February 20, 1991, discloses shampoo containing anionic surfactant, and conditioning agents such as insoluble silicone (preferably aminosilicone), cationic surfactant, polyethylenes, paraffins, microcrystalline waxes, $C_{18}$-$C_{36}$ fatty acids or triglycerides, high fatty alcohol esters of high fatty acids, and beeswax. Another patent document which discloses shampoo compositions and a variety of conditioning agents is US-A-3,964,500, Drakoff, issued June 22, 1976. This patent relates to shampoo containing silicone conditioner and a hair bodying agent selected from certain wood rosins, shellac, sucrose acetate isobutyrate, and cationic amino cellulose

In spite of all these approaches and attempts to provide optimum combinations of shampoos and hair conditioners, it remains desirable to provide still improved conditioning shampoos. It has now been found that improved overall conditioning can be found by combining anionic surfactant in a shampoo with insoluble, dispersed, nonionic silicone, a soluble cationic organic polymer hair conditioning agent, and specific organic oily liquids. These compositions can provide improved conditioning while reducing the level of undesirable side effects that can result from increasing the level of conditioning agent in prior known conditioning systems. As discussed previously, a conditioning agent system containing too much silicone can result in silicone build up on the hair over repeated usages and to loss of fullness of the hair. Too much oil results in an oily feel and a loss of fullness of the hair. Too much cationic conditioning agent results in a slick, oily feel of the hair. Now it has been found that combining these specific types of ingredients - insoluble nonionic silicones, oily organic liquids, and cationic polymers - can provide improved overall conditioning while minimizing the adverse effects of conditioning agent build-up that otherwise can be incurred upon Increasing the levels of individual components in prior known conditioning systems. Furthermore, the use of cationic polymer in the compositions hereof can improve performance relative to similar systems with cationic surfactant in combination with silicone and oily liquid conditioning agents.

It is an object of this invention to provide shampoo compositions, which can provide excellent cleaning performance and improved levels of conditioning while minimizing any adverse side effects associated with build-up due to the use of excess conditioning agent.

It is also an object of this invention to provide a method for cleaning and conditioning the hair which can provide excellent cleaning in combination with improved conditioning, while minimizing adverse side effects associated with excess build-up of conditioning agent on the hair.

These objects will become apparent from the description which follows, as may other objects become apparent upon a reading of said description.

This invention provides anionic detersive surfactant-containing liquid shampoo compositions that can provide both excellent cleaning performance and hair conditioning benefits to a wide variety of hair types. This can be attained by incorporating into the shampoo composition a tri-component hair conditioning system including a nonionic, insoluble, nonvolatile silicone hair conditioning agent within a specific range of cationic charge density, a water soluble, cationic polymer conditioning agent, and an organic, nonvolatile, water insoluble oily liquid. The shampoo compositions hereof also comprise an aqueous carrier.

The cationic polymer conditioning agents of the present invention are organic polymers having quaternary ammonium or amino moieties. The amino moieties are cationic at the pH of the shampoo composition, which is preferably between about pH 3 and about pH 9, more preferably from about pH 4 to about pH 8.

The insoluble silicone conditioning agent is dispersed throughout the composition in the form of droplets or particles. Preferably, a suitable suspending agent is utilized to facilitate stability of the dispersed silicone.

The oily liquid conditioning agent is intermixed in, and is distributed throughout, the composition. The oily liquid is generally selected from the group consisting of hydrocarbon oils and fatty esters. As used herein, "fatty ester" means esters having 10 or more carbon atoms.

More particularly, the present invention provides hair conditioning shampoo compositions comprising:

(a) from 5% to 50%, by weight, of an anionic surfactant component;

(b) from 0.05% to 10%, by weight, of a dispersed, insoluble, nonvolatile, nonionic silicone conditioning agent;

(c) from 0.05% to 5%, by weight, of water soluble, organic, cationic polymer hair conditioning agent having a cationic charge density of from 0.9 to 4 meq/gram;

(d) from 0.05% to 5%, by weight, of an organic, nonvolatile, water insoluble, liquid selected from the group consisting of hydrocarbon oils, fatty esters having 10 or more carbon atoms, and mixtures thereof; and

(e) an aqueous carrier.

As used herein, the terms "soluble" and "insoluble" used in reference to particular ingredients of the shampoo compositions refer to solubility or insolubility, respectively, of that ingredient in the shampoo composition, unless otherwise specifically indicated. For example the terms "water soluble" and "water insoluble", as used herein, refer to solubility of the particular ingredient in water, as opposed to solubility in the shampoo composition.

The invention, including preferred embodiments thereof, is described in further detail in the Detailed Description of the Invention, which follows.

The compositions of the present invention can comprise, consist essentially of, or consist of the various essential and/or optional ingredients described herein.

All percentages are calculated by weight of the total composition unless otherwise specifically indicated. All ratios are weight ratios unless otherwise specifically indicated.

The essential as well as a variety of preferred and optional ingredients of the compositions of the present invention are described below.

The hair conditioning shampoo compositions of the present invention contain an anionic surfactant component, which can comprise one or more anionic detersive surfactants or amphoteric detersive surfactants which are anionic at the pH of the shampoo, to provide cleaning performance to the composition.

The anionic surfactant component will generally be present at a level from 5% to 50%, preferably from 8% to 30%, more preferably from 10% to 25%, of the composition.

Anionic detersive detergents useful herein include alkyl and alkyl ether sulfates. These materials have the respective formulae $ROSO_3M$ and $RO(C_2H_4O)_xSO_3M$, wherein R is alkyl or alkenyl of from about 8 to about 24 carbon atoms, x is 1 to 10, and M is a water-soluble cation such as ammonium, sodium, potassium and triethanolamine. The alkyl ether sulfates are typically made as condensation products of ethylene oxide and monohydric alcohols having from about 8 to about 24 carbon atoms. Preferably, R has from about 12 to about 18 carbon atoms in both the alkyl and alkyl ether sulfates. The alcohols can be derived from fats, e.g., coconut oil or tallow, or can be synthetic. Lauryl alcohol and straight chain alcohols derived from coconut oil are preferred herein. Such alcohols are reacted with about 1 to about 10, and especially about 3, molar proportions of ethylene oxide and the resulting mixture of molecular species having, for example, an average of 3 moles of ethylene oxide per mole of alcohol, is sulfated and neutralized.

Specific examples of alkyl ether sulfates which may be used in the present invention are sodium and ammonium salts of coconut alkyl triethylene glycol ether sulfate; tallow alkyl triethylene glycol ether sulfate, and tallow alkyl hexaoxyethylene sulfate. Highly preferred alkyl ether sulfates are those comprising a mixture of individual compounds, said mixture having an average alkyl chain length of from about 12 to about 16 carbon atoms and an average degree of ethoxylation of from about 1 to about 4 moles of ethylene oxide. Such a mixture also comprises from 0 to 20% by weight $C_{12-13}$ compounds; from 60 to 100% by weight of $C_{14-15-16}$ compounds, from 0 to 20% by weight of $C_{17-18-19}$ compounds; from 3 to 30% by weight of compounds having a degree of ethoxylation of 0; from 45 to 90% by weight of compounds having a degree of ethoxylation of from about 1 to about 4; from 10 to 25% by weight of compounds having a degree of ethoxylation of from about 4 to about 8; and from 0.1 to 15% by weight of compounds having a degree of ethoxylation greater than about 8.

Another suitable class of anionic detersive surfactants are the water-soluble salts of the organic, sulfuric acid reaction products of the general formula:

$$R_1\text{-}SO_3\text{-}M$$

wherein $R_1$ is chosen from the group consisting of a straight or branched chain, saturated aliphatic hydrocarbon radical having from about 8 to about 24, preferably about 12 to about 18, carbon atoms; and M is a cation. Important examples are the salts of an organic sulfuric acid reaction product of a hydrocarbon of the methane series, including iso-, neo-, ineso-, and n-paraffins, having about 8 to about 24 carbon atoms, preferably about 12 to about 18 carbon atoms and a sulfonating agent, e.g., $SO_3$, $H_2SO_4$, oleum, obtained according to known sulfonation methods, including bleaching and hydrolysis. Preferred are alkali metal and ammonium sulfonated $C_{12-18}$ n-paraffins.

Additional examples of synthetic anionic detersive surfactants which come within the terms of the present invention are the reaction products of fatty acids esterified with isethionic acid and neutralized with sodium hydroxide where, for example, the fatty acids are derived from coconut oil; sodium or potassium salts of fatty acid amides of methyl tauride in which the fatty acids, for example, are derived from coconut oil. Other synthetic anionic detersive surfactants of this variety are set forth in US-A-2,486,921; 2,486,922; and US-A-2,396,278.

Still other synthetic anionic detersive surfactants are in the class designated as succinamates. This class includes such surface active agents as disodium N-octadecylsulfosuccinamate; tetrasodium N-(1,2-dicarboxyethyl)-N-octadecylsulfosuccinamate; diamyl ester of sodium sulfosuccinic acid; dihexyl ester of sodium sulfosuccinic acid; dioctyl esters of sodium sulfosuccinic acid.

Other suitable anionic detersive surfactants include olefin sulfonates having about 12 to about 24 carbon atoms. The term "olefin sulfonates" is used herein to mean compounds which can be produced by the sulfonation of α-olefins by means of uncomplexed sulfur trioxide, followed by neutralization of the acid reaction mixture in conditions such that any sulfones which have been formed in the reaction are hydrolyzed to give the corresponding hydroxy-alkanesulfonates. The sulfur trioxide can be liquid or gaseous, and is usually, but not necessarily, diluted by inert diluents, for example by liquid $SO_2$, chlorinated hydrocarbons, etc., when used in the liquid form, or by air, nitrogen, gaseous $SO_2$, etc., when used in the gaseous form.

The α-olefins from which the olefin sulfonates are derived are mono-olefins having about 12 to about 24 carbon atoms, preferably about 14 to about 16 carbon atoms. Preferably, they are straight chain olefins.

In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process.

A specific α-olefin sulfonate mixture of the above type is described more fully in the US-A-3,332,880, Pflaumer and Kessler, issued July 25, 1967.

Another class of anionic detersive surfactants are the β-alkyloxy alkane sulfonates. These compounds have the following formula:

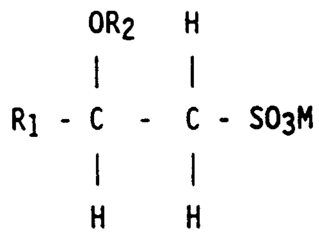

where $R_1$ is a straight chain alkyl group having from about 6 to about 20 carbon atoms, $R_2$ is a lower alkyl group having from about 1 (preferred) to about 3 carbon atoms, and M is a water-soluble cation as hereinbefore described.

Many additional synthetic anionic surfactants are described in McCutcheon's, Emulsifiers and Detergents, 1989 Annual, published by M. C. Publishing Co. Also US-A-3,929,678, Laughlin et al., issued December 30, 1975, discloses many other anionic as well as other surfactant types.

Preferred anionic detersive surfactants for use in the present shampoo compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium. sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, and sodium dodecyl benzene sulfonate.

Examples of amphoteric detersive surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent 2,658,072, N-higher alkyl aspartic acids such as those produced according to the teaching of US-A-2,438,091, and the products sold under the trade name "MIRANOL"™ (RTM) and described in US-A-2,528,378.

In addition to the anionic detersive surfactant component, the compositions of the present invention can optionally contain other detersive surfactants. These include nonionic surfactants, amphoteric surfactants that are not anionic at the pH of the shampoo, and zwitterionic surfactants. Optional detersive surfactants, when used, are typically present at levels of from 0.5% to 20%, more typically from 1% to 10% by weight, although higher or lower levels can be used. The total amount of detersive surfactant in compositions containing optional detersive surfactants in addition to the anionic

surfactant will generally be from 5.5% to 50%, preferably from 8% to 30%, more preferably from 10% to 25% by weight. Cationic detersive surfactants can also be used, but are generally less preferred because they can adversely interact with the anionic detersive surfactant. Cationic detersive surfactants, if used, are preferably used at levels no greater than 5% by weight. Cationic surfactants, if used, are more typically conditioning agents which can optionally be included in the compositions hereof.

Nonionic detersive surfactants which can be used include those broadly defined as compounds produced by the condensation of alkylene oxide groups (hydrophilic in nature) with an organic hydrophobic compound, which may be aliphatic or alkyl aromatic in nature. Examples of preferred classes of nonionic detersive surfactants are:

1. The polyethylene oxide condensates of alkyl phenols, e.g., the condensation products of alkyl phenols having an alkyl group containing from about 6 to about 20 carbon atoms in either a straight chain or branched chain configuration, with ethylene oxide, the said ethylene oxide being present in amounts equal to from about 10 to about 60 moles of ethylene oxide per mole of alkyl phenol.

2. Those derived from the condensation of ethylene oxide with the product resulting from the reaction of propylene oxide and ethylene diamine products.

3. The condensation product of aliphatic alcohols having from about 8 to about 18 carbon atoms, in either straight chain or branched chain configuration, with ethylene oxide, e.g., a coconut alcohol ethylene oxide condensate having from about 10 to about 30 moles of ethylene oxide per mole of coconut alcohol, the coconut alcohol fraction having from about 10 to about 14 carbon atoms.

4. Long chain tertiary amine oxides corresponding to the following general formula:

$$R_1R_2R_3N \rightarrow O$$

wherein $R_1$ contains an alkyl, alkenyl or monohydroxy alkyl radical of from about 8 to about 18 carbon atoms, from 0 to about 10 ethylene oxide moieties, and from 0 to about 1 glyceryl moiety, and $R_2$ and $R_3$ contain from about 1 to about 3 carbon atoms and from 0 to about 1 hydroxy group, e.g., methyl, ethyl, propyl, hydroxyethyl, or hydroxypropyl radicals. The arrow in the formula is a conventional representation of a semipolar bond.

5. Long chain tertiary phosphine oxides corresponding to the following general formula:

$$RR'R''P \rightarrow O$$

wherein R contains an alkyl, alkenyl or monohydroxyalkyl radical ranging from about 8 to about 18 carbon atoms in chain length, from 0 to about 10 ethylene oxide moieties and from 0 to about 1 glyceryl moiety and R' and R" are each alkyl or monohydroxyalkyl groups containing from about 1 to about 3 carbon atoms.

6. Long chain dialkyl sulfoxides containing one short chain alkyl or hydroxy alkyl radical of from about 1 to about 3 carbon atoms (usually methyl) and one long hydrophobic chain which include alkyl, alkenyl, hydroxy alkyl, or keto alkyl radicals containing from about 8 to about 20 carbon atoms, from 0 to about 10 ethylene oxide moieties and from 0 to about 1 glyceryl moiety.

7. Alkyl polysaccharide (APS) surfactants such as the alkyl polyglycosides. Such surfactants are described in US-A-4,565,647, Llenado, issued January 21, 1986, which discloses APS surfactants having a hydrophobic group with about 6 to about 30 carbon atoms and polysaccharide (e.g., polyglycoside) as the hydrophilic group. Optionally, there can be a polyalkylene-oxide group joining the hydrophobic and hydrophilic moieties. The alkyl group (i.e., the hydrophobic moiety) can be saturated or unsaturated, branched or unbranched, and unsubstituted or substituted (e.g., with hydroxy or cyclic rings).

8. Polyethylene glycol (PEG) glyceryl fatty esters, such as those of the formula $R(O)OCH_2CH(OH)CH_2(OCH_2CH_2)_nOH$ wherein n is from about 5 to about 200, preferably from about 20 to about 100, and R is an aliphatic hydrocarbyl having from about 8 to about 20 carbon atoms.

Zwitterionic detersive surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. A general formula for these compounds is:

$$\begin{array}{c} (R^3)_x \\ | \\ R^2 - Y^{(+)} - CH_2 - R^4 - Z^{(-)} \end{array}$$

wherein $R^2$ contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; $R^3$ is an alkyl or monohydroxyalkyl group containing about 1 to about 3 carbon atoms; X is 1 when Y is a sulfur atom, and 2 when Y is a nitrogen or phosphorus atom; $R^4$ is an alkylene or hydroxyalkylene of from about 1 to about 4 carbon atoms and Z is a radical selected from the group consisting of carboxylate, sulfonate, sulfate, phosphonate, and phosphate groups.

Other zwitterionics such as betaines can also useful in the present invention. Examples of betaines useful herein include the high alkyl betaines, such as coco dimethyl carboxymethyl betaine, cocoamidopropyl betaine, cocobetaine, lauryl amidopropyl betaine, oleyl betaine, lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, and lauryl bis-(2-hydroxypropyl)alpha-carboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like; amidobetaines and amidosulfobetaines, wherein the $RCONH(CH_2)_3$ radical is attached to the nitrogen atom of the betaine are also useful in this invention.

Preferred shampoos of the present invention contain combinations of anionic surfactants with zwitterionic surfactants and/or amphoteric surfactants. Especially preferred shampoos contain from 0% to 16% of alkyl sulfates, from 0% to 16% by weight of ethoxylated alkyl sulfates, and from 0% to 10% by weight of optional detersive surfactants selected from the nonionic, amphoteric, and zwitterionic detersive surfactants, with at least 5% by weight of either alkyl sulfate, ethoxylated alkyl sulfate, or a mixture thereof, and a total surfactant level of from 10% to 25% by weight.

An essential component of the present invention is a non-volatile, nonionic silicone hair conditioning agent which is insoluble in the shampoo compositions hereof. The silicone hair conditioning agent is intermixed in the shampoo composition so as to be in the form of dispersed, insoluble particles, or droplets. The silicone hair conditioning agent comprises a nonvolatile, insoluble, silicone fluid and optionally comprises a silicone gum which is insoluble in the shampoo composition as a whole but is soluble in the silicone fluid. The silicone hair conditioning agent can also comprise other ingredients, such as a silicone resin to enhance deposition efficiency.

The silicone hair conditioning agent may comprise low levels of volatile silicone components; however, such volatile silicones will preferably exceed no more than 0.5%, by weight, of the shampoo composition. Typically, if volatile silicones are present, it will be incidental to their use as a solvent or carrier for commercially available forms of other ingredients, such as silicone gums and resins.

The silicone hair conditioning agent for use herein will preferably have viscosity of from about 1,000 to about 2,000,000 $mm^2s^{-1}$ (centistokes) at 25°C, more preferably from about 10,000 to about 1,800,000, even more preferably from about 100,000 to about 1,500,000. The viscosity can be measured by means of a glass capillary viscometer as set forth in Dow Corning Corporate Test Method CTM0004, July 20, 1970.

The silicone hair conditioning agent will be used in the shampoo compositions hereof at levels of from 0.05% to 10% by weight of the composition, preferably from 0.1% to 10%, more preferably from 0.5% to 8%, most preferably from 0.5% to 5% by weight.

Suitable insoluble, nonvolatile silicone fluids include polyalkyl siloxanes, polyaryl siloxanes, polyalkylaryl siloxanes, polyether siloxane copolymers, and mixtures thereof. Other insoluble, nonvolatile silicone fluids having hair conditioning properties can also be used. The term "nonvolatile" as used herein shall mean that the silicone material exhibits very low or no significant vapor pressure at ambient conditions, as is understood by those in the art. The term "silicone fluid" shall mean flowable silicone materials having a viscosity of less than 1,000,000 $mm^2s^{-1}$ (centistokes) at 25°C. Generally, the viscosity of the fluid will be between about 5 and 1,000,000 $mm^2s^{-1}$ (centistokes) at 25°C, preferably between about 10 and about 100,000 $mm^2s^{-1}$.

Silicone fluids hereof also include polyalkyl or polyaryl siloxanes with the following structure:

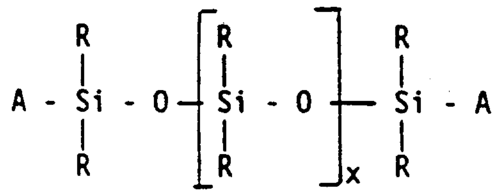

wherein R is alkyl or aryl, and x is an integer from about 7 to about 8,000 may be used. "A" represents groups which block the ends of the silicone chains.

The alkyl or aryl groups substituted on the siloxane chain (R) or at the ends of the siloxane chains (A) may have any structure as long as the resulting silicones remain fluid at room temperature, are hydrophobic, are neither irritating, toxic nor otherwise harmful when applied to the hair, are compatible with the other components of the composition, are chemically stable under normal use and storage conditions, and are capable of being deposited on and of conditioning hair.

Suitable A groups include methyl, methoxy, ethoxy, propoxy, and aryloxy. The two R groups on the silicone atom may represent the same group or different groups. Preferably, the two R groups represent the same group. Suitable R groups include methyl, ethyl, propyl, phenyl, methylphenyl and phenylmethyl. The preferred silicones are polydimethyl siloxane, polydiethylsiloxane, and polymethylphenylsiloxane. Polydimethylsiloxane is especially preferred.

The nonvolatile polyalkylsiloxane fluids that may be used include, for example, polydimethylsiloxanes. These siloxanes are available, for example, from the General Electric Company in their Viscasil® (RTM) and SF 96 series, and from Dow Corning in their Dow Corning 200 series.

The polyalkylaryl siloxane fluids that may be used, also include, for example, polymethylphenylsiloxanes. These siloxanes are available, for example, from the General Electric Company as SF 1075 methyl phenyl fluid or from Dow Corning as 556 Cosmetic Grade Fluid (RTM).

The polyether siloxane copolymers that may be used include, for example, a polypropylene oxide modified polydimethylsiloxane (e.g., Dow Corning DC-1248 (RTM)) although ethylene oxide or mixtures of ethylene oxide and propylene oxide may also be used. The ethylene oxide and polypropylene oxide level must be sufficiently low to prevent solubility in water and the composition hereof.

References disclosing suitable silicone fluids include US-A-2,826,551, Geen; US-A-3,964,500, Drakoff, issued June 22, 1976; US-A-4,364,837, Pader; and GB-A-849,433, Woolston. *Silicon Compounds* distributed by Petrarch Systems, Inc., 1984., provides an extensive (though not exclusive) listing of suitable silicone fluids.

Another silicone material that can be especially useful in the silicone conditioning agents is insoluble silicone gum. The term "silicone gum", as used herein, means polyorganosiloxane materials having a viscosity at 25°C of greater than or equal to 1,000,000 $mm^2s^{-1}$ (centistokes). Silicone gums are described by Petrarch and others including US-A-4,152,416, Spitzer et al., issued May 1, 1979 and Noll, Walter, *Chemistry and Technology of Silicones*, New York: Academic Press 1968. Also describing silicone gums are General Electric Silicone Rubber Product Data Sheets SE 30, SE 33, SE 54 and SE 76. The "silicone gums" will typically have a mass molecular weight in excess of about 200,000, generally between about 200,000 and about 1,000,000. Specific examples include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

Preferably the silicone hair conditioning agent comprises a mixture of a polydimethylsiloxane gum, having a viscosity greater than about 1,000,000 $mm^2s^{-1}$ (centistokes) and polydimethylsiloxane fluid having a viscosity of from about 10 $mm^2s^{-1}$ (centistokes) to about 100,000 $mm^2s^{-1}$ (centistokes), wherein the ratio of gum to fluid is from about 30:70 to about 70:30, preferably from about 40:60 to about 60:40.

Another optional ingredient that can be included in the silicone conditioning agent is silicone resin. Silicone resins are highly crosslinked polymeric siloxane systems. The crosslinking is introduced through the incorporation of trifunctional and tetrafunctional silanes with monofunctional or difunctional, or both, silanes during manufacture of the silicone resin. As is well understood in the art, the degree of crosslinking that is required in order to result in a silicone resin will vary according to the specific silane units incorporated into the silicone resin. In general, silicone materials which have a sufficient level of trifunctional and tetrafunctional siloxane monomer units (and hence, a sufficient level of crosslinking) such that they dry down to a rigid, or hard, film are considered to be silicone resins. The ratio of oxygen atoms to silicon atoms is indicative of the level of crosslinking in a particular silicone material. Silicone materials which have at least about 1.1 oxygen atoms per silicon atom will generally be silicone resins herein. Preferably, the ratio of oxygen:silicon atoms is at least about 1.2:1.0. Silanes used in the manufacture of silicone resins include monomethyl-, dimethyl-, trimethyl-, monophenyl-, diphenyl-, methylphenyl-, monovinyl-, and methylvinyl-chlorosilanes, and tetrachlorosilane, with the methyl-substituted silanes being most commonly utilized. Preferred resins are offered by General Electric as GE SS4230 (RTM) and SS4267 (RTM). Commercially available silicone resins will generally be supplied in a dissolved form

in a low viscosity volatile or nonvolatile silicone fluid. The silicone resins for use herein should be supplied and incorporated into the present compositions in such dissolved form, as will be readily apparent to those skilled in the art.

Background material on silicones including sections discussing silicone fluids, gums, and resins, as well as manufacture of silicones, can be found in *Encyclopedia of Polymer Science and Engineering*, Volume 15, Second Edition, pp 204-308, John Wiley & Sons, Inc., 1989.

Silicone materials and silicone resins in particular, can conveniently be identified according to a shorthand nomenclature system well known to those skilled in the art as "MDTQ" nomenclature. Under this system, the silicone is described according to presence of various siloxane monomer units which make up the silicone. Briefly, the symbol M denotes the monofunctional unit $(CH_3)_3SiO_{0.5}$; D denotes the difunctional unit $(CH_3)_2SiO$; T denotes the trifunctional unit $(CH_3)SiO_{1.5}$; and Q denotes the quadri- or tetra-functional unit $SiO_2$. Primes of the unit symbols, e.g., M', D', T', and Q' denote substituents other than methyl, and must be specifically defined for each occurrence. Typical alternate substituents include groups such as vinyl, phenyls, amines, hydroxyls, etc. The molar ratios of the various units, either in terms of subscripts to the symbols indicating the total number of each type of unit in the silicone (or an average thereof) or as specifically indicated ratios in combination with molecular weight complete the description of the silicone material under the MDTQ system. Higher relative molar amounts of T, Q, T' and/or Q' to D, D', M and/or or M' in a silicone resin is indicative of higher levels of crosslinking. As discussed before, however, the overall level of crosslinking can also be indicated by the oxygen to silicon ratio.

The silicone resins for use herein which are preferred are MQ, MT, MTQ, MQ and MDTQ resins. Thus, the preferred silicone substituent is methyl. Especially preferred are MQ resins wherein the M:Q ratio is from about 0.5:1.0 to about 1.5:1.0 and the average molecular weight of the resin is from about 1000 to about 10,000.

The weight ratio of the nonvolatile silicone fluid component to the silicone resin component, when used, is from about 4:1 to about 400:1, preferably this ratio is from about 9:1 to about 200:1, more preferably from about 19:1 to about 100:1, particularly when the silicone fluid component is a polydimethylsiloxane fluid or a mixture of polydimethylsiloxane fluid and polydimethylsiloxane gum as described above.

The shampoo compositions of the present invention comprise a water soluble, cationic organic polymer hair conditioning agent as an essential element. The polymeric cationic hair conditioning agent hereof will generally be present at levels of from 0.05% to 5%, preferably from 0.1% to 4%, more preferably from 0.2% to 3%, by weight, of the shampoo composition. By "water soluble" cationic organic polymer, what is meant is a polymer which is sufficiently soluble in water to form a substantially clear solution to the naked eye at a concentration of 0.1% in water (distilled or equivalent) at 25°C. Preferably, the polymer will be sufficiently soluble to form a substantially clear solution at 0.5% concentration, more preferably at 1.0% concentration.

The cationic organic polymers useful in the hair conditioning agent hereof are organic polymers that can provide conditioning benefits to hair and that are soluble in the shampoo composition. Any cationic polymers which can provide these benefits can be used. As used herein, the term "polymer" shall include materials whether made by polymerization of one type of monomer or made by two (i.e., copolymers) or more types of monomers.

The cationic polymers hereof will generally have a weight average molecular weight which is at least about 5,000, typically at least about 10,000, and is less than about 10 million. Preferably, the molecular weight is from about 100,000 to about 2 million. The cationic polymers will have cationic nitrogen-containing moieties such as quaternary ammonium or cationic amino moieties, or a mixture thereof.

The cationic charge density is at least about 0.9 meq/gram, more preferably at least about 1.0 meq/gram, even more preferably at least abut 1.1 meq/gram, most preferably at least about 1.2 meq/gram. The cationic charge density should also generally be no greater than about 4 meq/gram, more preferably no greater than about 3.0 meq/gram, most preferably no greater than about 2.0 meq/gram. Cationic charge density of the cationic polymer can be determined according to the Kjeldahl Method. Those skilled in the art will recognize that the charge density of amino-containing polymers may vary depending upon pH and the isoelectric point of the amino groups. The charge density should be within the above limits at the pH of intended use, which will in general be from about pH 3 to about pH 9, most generally from about pH 4 to about pH 8.

Any anionic counterions can be utilized for the cationic polymers so long as the water solubility criteria is met. Suitable counterions include halides (e.g., Cl, Br, I, or F, preferably Cl, Br, or I), sulfate, and methylsulfate. Others can also be used, as this list is not exclusive.

The cationic nitrogen-containing moiety will be present generally as a substituent, on a fraction of the total monomer units of the cationic hair conditioning polymers. Thus, the cationic polymer can comprise copolymers, terpolymers, etc. of quaternary ammonium or cationic amine-substituted monomer units and other non-cationic units referred to herein as spacer monomer units. Such polymers are known in the art, and a variety can be found in the *CTFA Cosmetic Ingredient Dictionary*, 3rd edition, edited by Estrin, Crosley, and Haynes, (The Cosmetic, Toiletry, and Fragrance Association, Inc., Washington, D.C., 1982).

Suitable cationic polymers include, for example, copolymers of vinyl monomers having cationic amine or quaternary ammonium functionalities with water soluble spacer monomers such as acrylamide, methacrylamide, alkyl and dialkyl acrylamides, alkyl and dialkyl methacrylamides, alkyl acrylate, alkyl methacrylate, vinyl caprolactone, and vinyl

pyrrolidone. The alkyl and dialkyl substituted monomers preferably have $C_1$-$C_7$ alkyl groups, more preferably $C_1$-$C_3$ alkyl groups. Other suitable spacer monomers include vinyl esters, vinyl alcohol (made by hydrolysis of polyvinyl acetate), maleic anhydride, propylene glycol, and ethylene glycol.

The cationic amines can be primary, secondary, or tertiary amines, depending upon the particular species and the pH of the shampoo. In general, secondary and tertiary amines, especially tertiary amines, are preferred.

Amine-substituted vinyl monomers can be polymerized in the amine form, and then optionally can be converted to ammonium by a quaternization reaction. Amines can also be similarly quaternized subsequent to formation of the polymer. For example, tertiary amine functionalities can be quaternized by reaction with a salt of the formula R'X wherein R' is a short chain alkyl, preferably a $C_1$-$C_7$ alkyl, more preferably a $C_1$-$C_3$ alkyl, and X is an anion which forms a water soluble salt with the quaternized ammonium.

Suitable cationic amino and quaternary ammonium monomers include, for example, vinyl compounds substituted with dialkylaminoalkyl acrylate, dialkylaminoalkyl methacrylate, monoalkylaminoalkyl acrylate, monoalkylaminoalkyl methacrylate, trialkyl methacryloxyalkyl ammonium salt, trialkyl acryloxyalkyl ammonium salt, diallyl quaternary ammonium salts, and vinyl quaternary ammonium monomers having cyclic cationic nitrogen-containing rings such as pyridinium, imidazolium, and quaternized pyrrolidone, e.g., alkyl vinyl imidazolium, alkyl vinyl pyridinium, alkyl vinyl pyrrolidone salts. The alkyl portions of these monomers are preferably lower alkyls such as the $C_1$-$C_3$ alkyls, more preferably $C_1$ and $C_2$ alkyls.

Suitable amine-substituted vinyl monomers for use herein include dialkylaminoalkyl acrylate, dialkylaminoalkyl methacrylate, dialkylaminoalkyl acrylamide, and dialkylaminoalkyl methacrylamide, wherein the alkyl groups are preferably $C_1$-$C_7$ hydrocarbyls, more preferably $C_1$-$C_3$, alkyls.

The cationic polymers hereof can comprise mixtures of monomer units derived from amine- and/or quaternary ammonium-substituted monomer and/or compatible spacer monomers.

Suitable cationic hair conditioning polymers include, for example: copolymers of 1-vinyl-2-pyrrolidone and 1-vinyl-3-methyl-imidazolium salt (e.g., chloride salt) (referred to in the industry by the Cosmetic, Toiletry, and Fragrance Association, "CTFA", as Polyquaternium-16), such as those commercially available from BASF Wyandotte Corp. (Parsippany, NJ, USA) under the LUVIQUAT (RTM) tradename (e.g., LUVIQUAT FC 370 (RTM)); copolymers of 1-vinyl-2-pyrrolidone and dimethylaminoethyl methacrylate (referred to in the industry by CTFA as Polyquaternium-11) such as those commercially available from Gaf Corporation (Wayne, NJ, USA) under the GAFQUAT tradename (e.g., GAFQUAT 755N (RTM)); cationic diallyl quaternary ammonium-containing polymers, including, for example, dimethyldiallylammonium chloride homopolymer and copolymers of acrylamide and dimethyldiallylammonium chloride, referred to in the industry (CTFA) as Polyquaternium 6 and Polyquaternium 7, respectively; and mineral acid salts of amino-alkyl esters of homo- and co-polymers of unsaturated carboxylic acids having from 3 to 5 carbon atoms, as described in US-A-4,009,256.

Other cationic polymers that can be used include polysaccharide polymers, such as cationic cellulose derivatives and cationic starch derivatives.

Cationic polysaccharide polymer materials suitable for use herein include those of the formula:

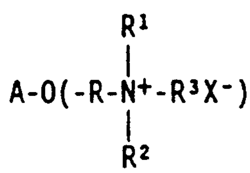

wherein:

A is an anhydroglucose residual group, such as a starch or cellulose anhydroglucose residual,

R is an alkylene oxyalkylene, polyoxyalkylene, or hydroxyalkylene group, or combination thereof,

$R^1$, $R^2$, and $R^3$ independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in $R^1$, $R^2$ and $R^3$) preferably being about 20 or less, and

X is an anionic counterion, as previously described.

Cationic cellulose is available from Amerchol Corp. (Edison, NJ, USA) in their Polymer JR® (RTM) and LR® (RTM) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted opoxide, referred to in the industry (CTFA) as Polyquaternium 24. These materials are-available from Amerchol Corp. (Edison, NJ, USA) under the trade-name Polymer LM-200 (RTM).

Other cationic polymers that can be used include cationic guar gum derivatives, such as guar hydroxypropyltrimonium chloride (commercially available from Celanese Corp. in their Jaguar® (RTM) series). Other materials include

quaternary nitrogen-containing cellulose ethers (e.g., as described in US-A-3,962,418, incorporated by reference herein), and copolymers of etherified cellulose and starch (e.g., as described in US-A-3,958,581).

As discussed above, the cationic polymer hereof is water soluble. This does not mean, however, that it must be soluble in the shampoo composition. Preferably however, the cationic polymer is either soluble in the shampoo composition, or in a complex coacervate phase in the shampoo composition formed by the cationic polymer and anionic material. Complex coacervates of the cationic polymer can be formed with anionic surfactants or with anionic polymers that can optionally be added to the compositions hereof (e.g., sodium polystyrene sulfonate).

Coacervate formation is dependent upon a variety of criteria such as molecular weight, concentration, and ratio of interacting ionic materials, ionic strength (including modification of ionic strength, for example, by addition of salts), charge density of the cationic and anionic species, pH, and temperature. Coacervate systems and the effect of these parameters has previously been studied. See, for example, J. Caelles, et al., "Anionic and Cationic Compounds in Mixed Systems", *Cosmetics & Toiletries*, Vol. 106, April 1991, pp 49-54, C. J. van Oss, "Coacervation, Complex-Coacervation and Flocculation", *J. Dispersion Science and Technology*, Vol. 9 (5,6), 1988-89, pp 561-573, and D. J. Burgess, "Practical Analysis of Complex Coacervate Systems", *J. of Colloid and Interface Science*, Vol. 140, No. 1, November 1990, pp 227-238.

It is believed to be particularly advantageous for the cationic polymer to be present in the shampoo in a coacervate phase, or to form a coacervate phase upon application or rinsing of the shampoo to or from the hair. Complex coacervates are believed to more readily deposit on the hair. Thus, in general, it is preferred that the cationic polymer exist in the shampoo as a coacervate phase or form a coacervate phase upon dilution. If not already a coacervate in the shampoo, the cationic polymer will preferably exist in a complex coacervate form in the shampoo upon dilution with water to a water: shampoo composition weight ratio of about 20:1, more preferably at about 10:1, even more preferably at about 8:1.

Techniques for analysis of formation of complex coacervates are known in the art. For example, microscopic analyses of the shampoo compositions, at any chosen stage of dilution, can be utilized to identify whether a coacervate phase has formed. Such coacervate phase will be identifiable as an additional emulsified phase in the composition. The use of dyes can aid in distinguishing the coacervate phase from other insoluble phases dispersed in the composition.

Exemplary complex coacervate shampoo compositions are shown in the examples. Many other cationic polymers, depending upon the other parameters of the shampoo composition, can also form coacervates, as will be understood by those skilled In the art.

It has been found that for compositions containing silicone and a conditioning oily liquid (as described below), cationic polymer conditioning agents having sufficiently high cationic charge density within the above range can provide enhanced conditioning performance and coacervate formation.

The shampoo compositions of the present invention comprise a nonvolatile, water insoluble, organic, oily liquid as a third essential type of hair conditioning agent. The hair conditioning oily liquid can add shine and luster to the hair. Additionally, it can also enhance dry `combing and dry hair feel. The hair conditioning oily liquid is typically present in the compositions at a level of from 0.05% to 5%, by weight of the composition, preferably from 0.2% to 3%, more preferably from 0.5% to 1%.

By "nonvolatile" what is meant is that the oily material exhibits very low or no significant vapor pressure at ambient conditions (e.g., 1 atmosphere, 25°C), as is understood in the art. The nonvolatile oily materials preferably have a boiling point at ambient pressure of about 250°C or higher.

By "water insoluble" what is meant is that the oily liquid is not soluble in water (distilled or equivalent) at a concentration of 0.1%, at 25°C.

The hair conditioning oily liquids hereof generally will have a viscosity of about 3 million $mm^2s^{-1}$ (cs) or less, preferably about 2 million $mm^2s^{-1}$ (cs) or less, more preferably about 1.5 million $mm^2s^{-1}$ (cs) or less.

The hair conditioning oily materials hereof are liquids selected from the group consisting of hydrocarbon oils and fatty esters. The fatty esters hereof are characterized by having at least 10 carbon atoms, and include esters with hydrocarbyl chains derived from fatty acids or alcohols, e.g., mono-esters, and di- and tri-carboxylic acid esters. The hydrocarbyl radicals of the fatty esters hereof can also include or have covalently bonded thereto other compatible functionalities, such as amides and alkoxy moieties (e.g., ethoxy or ether linkages, etc.).

Hydrocarbon oils include cyclic hydrocarbons, straight chain aliphatic hydrocarbons (saturated or unsaturated), and branched chain aliphatic hydrocarbons (saturated or unsaturated). Straight chain hydrocarbon oils will preferably contain from about 12 to about 19 carbon atoms, although it is not necessarily meant to be limit the hydrocarbons to this range. Branched chain hydrocarbon oils can and typically may contain higher numbers of carbon atoms. Also encompassed herein are polymeric hydrocarbons of alkenyl monomers, such as $C_2$-$C_6$ alkenyl monomers. These polymers can be straight or branched chain polymers. The straight chain polymers will typically be relatively short in length, having a total number of carbon atoms as described above for straight chain hydrocarbons in general. The branched chain polymers can have substantially higher chain length. The number average molecular weight of such materials can vary widely, but will typically be up to about 500, preferably from about 200 to about 400, more preferably from about 300 to about 350. Specific examples of suitable materials include paraffin oil, mineral oil, saturated and unsaturated

dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used. Exemplary branched-chain isomers are highly branched saturated or unsaturated alkanes, such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, sold by Permethyl Corporation. A preferred hydrocarbon polymer is polybutene, such as the copolymer of isobutylene and butene. A commercially available material of this type is L-14 polybutene from Amoco Chemical Co. (Chicago, Illinois, U.S.A.).

Monocarboxylic acid esters hereof inlude esters of alcohols and/or acids of the formula R'COOR wherein alkyl or alkenyl radicals and the sum of carbon atoms in R' and R is at least 10, preferably at least 20.

Fatty esters include, for example, alkyl and alkenyl esters of fatty acids having aliphatic chains with from about 10 to about 22 carbon atoms, and alkyl and alkenyl fatty alcohol carboxylic acid esters having an alkyl and/or alkenyl alcohol-derived aliphatic chain with about 10 to about 22 carbon atoms, and combinations thereof. Examples include isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

The mono-carboxylic acid ester however need not necessarily contain at least one chain with at least 10 carbon atoms, so long as the total number of aliphatic chain carbon atoms is at least 10. Examples include diisopropyl adipate, diisohexyl adipate, and diisopropyl sebacate.

Di- and tri-alkyl and alkenyl esters of carboxylic acids can also be used. These include, for example, esters of $C_4$-$C_8$ dicarboxylic acids such as $C_1$-$C_{22}$ esters (preferably $C_1$-$C_6$) of succinic acid, glutaric acid, adipic acid, hexanoic acid, heptanoic acid, and octanoic acid. Specific examples include isocetyl stearyol stearate, diisopropyl adipate, and tristearyl citrate.

Glycerides include mono-, di-, and tri-glycerides. More specifically, included are the mono-, di-, and tri-esters of glycerol and long chain carboxylic acids, such as $C_{10}$-$C_{22}$ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include triolein and tristearin glyceryl dilaurate. Preferred glycerides are di-, and tri-glycerides. Especially preferred are triglycerides.

The shampoo compositions of the present invention are typically liquids which, preferably, are pourable at room temperature. The compositions hereof will comprise an aqueous carrier, i.e., water, which will generally be present at a level of 20% to 95% by weight of the composition, preferably from 60% to 85% for pourable, liquid formulations. The compositions of the present invention can also be in other forms, such as gels, mouse, etc. In such cases, appropriate components known in the art such as gelling agents (e.g., hydroxyethyl cellulose), etc. can be included in the compositions. Gels will typically contain from 20% to 90% water. Mousses will be a low viscosity composition and will be packaged as a sprayable liquid according to techniques well known in the art, typically in an aerosol cannister including a propellant or a means for generating an aerosol spray.

Since the silicone conditioning agent used in the present compositions is an insoluble silicone dispersed in the compositions, it is preferred to utilize a suspending agent for the silicone. Suitable suspending agents are long chain acyl derivatives, long chain amine oxides, and mixtures thereof, wherein such suspending agents are present in the shampoo compositions in crystalline form. A variety of such suspending agents are described in US-A-4,741,855, Grote et al., issued May 3, 1988. Especially preferred is ethylene glycol distearate.

Also included among the long chain acyl derivatives useful as suspending agents are the N,N-di(hydrogenated) $C_8$-$C_{22}$ (preferably $C_{12}$-$C_{22}$, more preferably $C_{16}$-$C_{18}$) amido benzoic acid, or soluble salt (e.g., K, Na salts) thereof particularly N,N-di(hydrogenated)tallow amido benzoic acid which is commercially marketed by Stepan Company (Northfield, Illinois, USA).

Another useful suspending agent for the silicone conditioning agents of the present compositions is xanthan gum as described in US-A-4,788,006, Bolich et al., issued June 5, 1984. The combination of long chain acyl derivatives and xanthan gum as a suspending system for silicone is described in US-A-4,704,272, Oh et al., issued November 3, 1987, and may also be used in the present compostions.

Generally, the shampoo compositions will comprise from 0.1% to 5.0%, preferably from 0.5% to 3.0% by weight, of the suspending agent to suspend the silicone conditioning agent.

The present compositions may also comprise a variety of non-essential, optional shampoo components suitable for rendering such compositions more cosmetically or aesthetically acceptable or to provide them with additional usage benefits. A variety of such ingredients are well-known to those skilled in the art, and these include without limiting the invention thereto: pearlescent aids, such as coated mica, ethylene glycol distearate; opacifiers, such as $TiO_2$; preservatives, such as benzyl alcohol, 1,3-bis(hydroxymethyl)-5,5-dimethyl-2,3-imidazolidinedione (e.g., Glydant® (RTM), Glyco, Inc., Greenwich, CT, USA), methylchloroisothiazolinone (e.g., Kathon® (RTM), Rohm & Haas Co., Philadelphia, PA, USA), methyl paraben, propyl paraben, and imidazolidinyl urea; fatty alcohols, such as cetearyl alcohol, cetyl alcohol, and stearyl alcohol; sodium chloride; ammonium chloride; sodium sulfate; ethyl alcohol; pH adjusting aids, such as

citric acid, sodium citrate, succinic acid, phosphoric acid, monosodium phosphate, disodium phosphate, sodium hydroxide, and sodium carbonate; coloring agents or dyes; perfumes; and sequestering agents, such as disodium ethylenediamine tetra-acetate.

Another optional ingredient that can be advantageously used is an anti-static agent. The anti-static agent should not unduly interfere with the in-use performance and end-benefits of the shampoo; particularly, the anti-static agent should not interfere with the anionic detersive surfactant. Suitable anti-static agents include, for example, tricetyl methyl ammonium chloride.

Typically, from 0.1% to 5% by weight of such anti-static agent is incorporated into the shampoo compositions.

Though the silicone suspending agent component may act to thicken the present compositions to some degree, the present compositions may also optionally contain other thickeners and viscosity modifiers such as an ethanolamide of a long chain fatty acid (e.g., polyethylene (3) glycol lauramide and coconut monoethanolamide) and ammonium xylene sulfonate.

These optional components generally are used individually in the compositions of the present invention at a level of from 0.01% to 10%, preferably from 0.05% to 5.0% by weight of the shampoo composition.

The compositions of the present invention, in general, can be made by mixing the materials together at elevated temperature, e.g., about 72°C. The silicone resin, if any, and silicone fluid component are first mixed together before being mixed with the other ingredients. The other ingredients are added and the complete mixture is mixed thoroughly at the elevated temperature and is then pumped through a high shear mill and then through a heat exchanger to cool it to ambient temperature. The average particle size of the silicone is preferably from 0.5 microns to 20 microns. Alternately, the silicone conditioning agent can be mixed with anionic surfactant and fatty alcohol, such as cetyl and stearyl alcohols, at elevated temperature, to form a premix containing dispersed silicone. The premix can then be added to and mixed with the remaining materials of the shampoo, pumped through a high shear mill, and cooled. Also alternately, a portion of the liquid components or soluble components (including, for example, cationic polymer conditioning agent) can be added to the composition after cooling the mix of surfactants and solids.

The shampoo compositions of the present invention are utilized conventionally, i.e., the hair is shampooed by applying an effective amount of the shampoo composition to the scalp, and then rinsing it out with water. Application of the shampoo to the scalp in general, encompasses massaging or working the shampoo in the hair such that all or most of the hair on the scalp is contacted. The term an "effective amount" as used herein, is an amount which is effective in cleaning and conditioning the hair. Generally, from 1 g to 20 g of the composition is applied for cleaning and conditioning the hair. Preferably, the shampoo is applied to hair in a wet or damp state.

The compositions hereof can also be useful for cleaning and conditioning the skin. For such applications, the composition would be applied to the skin in a conventional manner, such as by rubbing or massaging the skin with the composition, optionally in the presence of water, and then rinsing it away with water.

## EXAMPLES

The following examples illustrate the present invention.

All parts, percentages, and ratios herein are by weight unless otherwise specified. Some components may come from suppliers as dilute solutions. The levels given reflect the weight percent of the active material, unless otherwise specified. The excluded diluents and other materials are included in as "Minors".

### Example I

The following is a shampoo composition of the present invention.

| Component | Weight % |
|---|---|
| Ammonium Lauryl Sulfate | 8.5 |
| Ammonium Laureth (3) Sulfate | 8.5 |
| JAGUAR C-17 [1] (RTM) | 0.5 |
| Coconut Monoethanol Amide | 1.0 |
| Ethylene Glycol Distearate | 2.0 |
| Isocetyl Stearoyl Stearate | 1.0 |
| Tricetyl Methyl Ammonium Chloride | 0.5 |
| Polydimethylsiloxane [2] | 2.0 |
| Cetyl Alcohol | 0.4 |
| Stearyl Alcohol | 0.2 |
| Perfume | 1.0 |
| Color Solution | 0.6 |
| Preservative | 0.4 |
| Water and Minors | -- to 100% -- |

[1] Tradename for guar hydroxypropyltrimonium chloride, a cationic polymer available from Rhone-Poulenc (Cranbury, NJ, USA).
[2] A 40/60 weight ratio blend of polydimethylsiloxane gum (GE SE 76, available from General Electric Co., Silicone Products Div., Waterford, NY, USA) and polydimethylsiloxane fluid (about 350 $mm^2s^{-1}$ (centistokes)).

The composition can provide excellent in-use hair cleaning and conditioning. As an alternative, the JAGUAR C-17 (RTM) can be replaced with LUVIQUAT FC 370 (RTM) (see Example III, footnote 1).

Example II

The following is an example of a shampoo composition of the present invention.

| Component | Weight % |
|---|---|
| Ammonium Lauryl Sulfate | 4.2 |
| Ammonium Laureth (3) Sulfate | 13.2 |
| POLYMER LR 400 [1] (RTM) | 1.0 |
| Coconut Monoethanol Amide | 1.0 |
| Ethylene Glycol Distearate | 2.0 |
| Light Mineral Oil | 1.0 |
| Tricetyl Methyl Ammonium Chloride | 0.5 |
| Polydimethylsiloxane [2] | 1.5 |
| Cetyl Alcohol | 0.4 |
| Stearyl Alcohol | 0.2 |
| Perfume | 1.2 |
| Color Solution | 0.6 |
| Preservative | 0.4 |
| Water and Minors | -- to 100% -- |

[1] Cellulose, 2-[2-hydroxy-3-(trimethyl ammonio)propoxy] ethyl ether, chloride, a cationic polymer available from Amerchol Corp. (Edison, NJ, USA).
[2] A 40/60 weight ratio blend of polydimethylsiloxane gum (GE SE 76, available from General Electric Co., Silicone Products Div., Waterford, NY, USA) and polydimethylsiloxane fluid (about 350 $mm^2s^{-1}$ (centistokes)).

The composition can provide excellent in-use hair cleaning and conditioning

Example III

The following is an example of a shampoo composition of the present invention wherein the cationic polymer and anionic surfactant component form a complex coacervate phase.

| Component | Weight % |
|---|---|
| Ammonium Lauryl Sulfate | 13.5 |
| Ammonium Laureth (3) Sulfate | 4.0 |
| LUVIQUAT FC 370 [1] (RTM) | 0.5 |
| Coconut Monoethanol Amide | 1.0 |
| Ethylene Glycol Distearate | 2.0 |
| Light Mineral Oil | 0.5 |
| Tricetyl Methyl Ammonium Chloride | 0.5 |
| Polydimethylsiloxane [2] | 3.0 |
| Cetyl Alcohol | 0.4 |
| Stearyl Alcohol | 0.2 |
| Perfume | 1.0 |
| Color Solution | 0.6 |
| Preservative | 0.4 |
| Water and Minors | 73.8 |

[1] Tradename of BASF Wyandotte Corporation (Parsippany, NJ, USA) for copolymer of vinyl pyrrolidone and methyl vinyl imidazolium chloride.
[2] A 40/60 weight ratio blend of polydimethylsiloxane gum (GE SE 76, available from General Electric Co., Silicone Products Div., Waterford, NY, USA) and polydimethylsiloxane fluid (about 350 $mm^2s^{-1}$ (centistokes)).

The composition can provide excellent in-use hair cleaning and conditioning. As an alternative, the LUVIQUAT FC 370 (RTM) can be replaced with JAGUAR C-17 (RTM) (see Example I, footnote 1).

Example IV

The following is an example of a shampoo composition of the present invention.

| Component | Weight % |
|---|---|
| Cocoamidopropyl Betaine | 4.0 |
| Ammonium Laureth (3) Sulfate | 12.0 |
| Coconut Monoethanol Amide | 2.0 |
| Ethylene Glycol Distearate | 2.0 |
| Polymer JR-125 [1] (RTM) | 1.0 |
| Isopropyl Isostearate | 1.0 |
| Tricetyl Methyl Ammonium Chloride | 0.5 |
| Polydimethylsiloxane [2] | 1.5 |
| Cetyl Alcohol | 0.4 |
| Stearyl Alcohol | 0.2 |
| Perfume | 1.0 |
| Color Solution | 0.6 |
| Preservative | 0.4 |
| Water and Minors | -- to 100% -- |

[1] Cellulose, 2-[2-hydroxy-3-(trimethyl ammonio)propoxy] ethyl ether, chloride, available from Amerchol Corp. (Edison, NJ, USA).

[2] VISCASIL (RTM) 12,500 $mm^2s^{-1}$ (cS) silicone fluid, available from General Electric (Waterford, NY, USA).

Example V

The following is a shampoo composition of the present invention.

| Component | Weight % |
|---|---|
| Ammonium Lauryl Sulfate | 13.5 |
| Ammonium Laureth (3) Sulfate | 4.0 |
| Polymer LM-200 [1] (RTM) | 1.0 |
| Light Mineral Oil | 1.0 |
| Coconut Monoethanol Amide | 1.0 |
| Ethylene Glycol Distearate | 2.0 |
| Tricetyl Methyl Ammonium Chloride | 0.5 |
| Polydimethylsiloxane [2] | 3.0 |
| Cetyl Alcohol | 0.4 |
| Stearyl Alcohol | 0.2 |
| Perfume | 1.0 |
| Color Solution | 0.6 |
| Preservative | 0.4 |
| Water and Minors | --- to 100% --- |

[1] Polyquaternium 24, a polymeric quaternary ammonium salt of hydroxyethyl cellulose reacted with lauryl dimethyl ammonium-substituted epoxide, available from Amerchol Corp. (Edison, NJ, USA).
[2] A 40/60 weight ratio blend of polydimethylsiloxane gum (GE SE 76, available from General Electric Co., Silicone Products Div., Waterford, NY, USA) and polydimethylsiloxane fluid (about 350 $mm^2s^{-1}$ (centistokes)).

Example VI

The following is a shampoo composition of the present invention wherein the cationic polymer and anionic surfactant component form a complex coacervate phase.

| Component | Weight % |
|---|---|
| Ammonium Lauryl Sulfate | 8.5 |
| Ammonium Laureth (3) Sulfate | 8.5 |
| GAFQUAT 755N[1] (RTM) | 0.5 |
| FLEXAN 130 [3] (RTM) | 0.5 |
| Coconut Monoethanol Amide | 1.0 |
| Ethylene Glycol Distearate | 2.0 |
| Isocetyl Stearoyl Stearate | 1.0 |
| Tricetyl Methyl Ammonium Chloride | 0.5 |
| Polydimethylsiloxane [2] | 2.0 |
| Cetyl Alcohol | 0.4 |
| Stearyl Alcohol | 0.2 |
| Perfume | 1.0 |
| Color Solution | 0.6 |
| Preservative | 0.4 |
| Water and Minors | -- to 100% -- |

[1] Copolymer of 1-vinyl-2-pyrrolidone and dimethylaminoethylmethacrylate, available from GAF Corp., Wayne, NJ, USA.
[2] VISCASIL (RTM), 600,000 $mm^2s^{-1}$ (cS), from General Electric, Waterford, NY, USA.
[3] Sodium polystyrene sulfonate, an anionic polymer available from National Starch and Chemical Corp., Bridgewater, NJ, USA.

The composition can provide excellent in-use hair cleaning and conditioning.

The example compositions hereof can be made by preparing a premix of the entire amount of silicone conditioning agent to be incorporated into the shampoo, along with sufficient ammonium sulfate and cetyl and stearyl alcohol such that the premix comprises about 30% silicone conditioning agent, about 69% surfactant, and about 1% of the alcohols. The premix ingredients are heated and stirred at 72°C for about 10 minutes and the premix is then conventionally mixed with the remaining hot (72°C) ingredients. The composition is then pumped through a high shear mixer and cooled.

## Claims

**1.** A hair conditioning shampoo composition characterized in that it comprises:

(a) from 5% to 50%, by weight, of an anionic surfactant component selected from the group consisting of anionic surfactants and amphoteric surfactants that are anionic at the pH of the composition;
(b) from 0.05% to 10%, by weight, of a dispersed, insoluble, nonvolatile, nonionic silicone hair conditioning agent;
(c) from 0.05% to 5%, by weight, of a water soluble, organic, cationic polymer hair conditioning agent having a cationic charge density of from 0.9 meq/gram to 4 meq/gram;
(d) from 0.05% to 5%, by weight, of an organic, nonvolatile, water insoluble, liquid selected from the group consisting of hydrocarbon oils, fatty esters having at least 10 carbon atoms selected from the group consisting of alkyl and alkenyl esters of fatty acids, alkyl and alkenyl esters of fatty alcohols, dicarboxylic acid esters, tricarboxylic acid esters, and mono-, di-, and triglycerides, and mixtures thereof; and
(e) an aqueous carrier.

2. A hair conditioning shampoo according to Claim 1, wherein said cationic charge density is from 1.0 meq/gram to 3.0 meq/gram, preferably from 1.1 meq/gram to 2.0 meq/gram, more preferably from 1.2 meq/gram to 2.0 meq/gram.

3. A hair conditioning shampoo composition as in Claim 1 or 2, further comprising a suspending agent for said silicone hair conditioning agent.

4. A hair conditioning shampoo composition, as in Claim 1, 2 or 3, wherein said cationic polymer is in a complex coacervate phase in said composition or forms a complex coacervate upon dilution of said shampoo with water.

5. A shampoo composition as in Claim 4 wherein said cationic polymer exists in a complex coacervate form upon dilution of the shampoo with water at a water: shampoo composition weight ratio of 20:1.

6. A shampoo composition as in Claim 1, 2, 3, 4 or 5 wherein said silicone hair conditioning agent comprises polydimethylsiloxane.

7. A shampoo composition as in Claim 1, 2, 3, 4, 5 or 6, wherein said cationic polymer hair conditioning agent is selected from the group consisting of cationic polysaccharides and polymers comprising cationic vinyl monomers, preferably said cationic polymer hair conditioning agent is selected from the group consisting of cationic cellulose, cationic starch, cationic guar, copolymers of 1-vinyl-2-pyrrolidone and 1-vinyl-3-methyl imidazolium salt, copolymers of acrylamide and dimethyl diallylammonium salt, and copolymers of 1-vinyl-2-pyrrolidone and dimethylaminoethyl methacrylate.

8. A shampoo composition as in Claim 1, 2, 3, 4, 5, 6 or 7, comprising from about 10% to about 25% of anionic surfactants, from about 0.5% to about 5% silicone hair conditioning agent, from about 0.1% to about 4% cationic polymer hair conditioning agent, and from about 0.2% to about 3% oily liquid hair conditioning agent.

9. A method for shampooing hair, said method comprising applying to hair an effective amount of the composition of Claim 1, 2, 3, 4, 5, 6, 7 or 8, for cleaning and conditioning the hair and then rinsing said composition from the hair.

## Patentansprüche

1. Haarkonditionierende Shampoozusammensetzung, dadurch gekennzeichnet, daß sie

   (a) 5 Gew.-% bis 50 Gew.-% von einer anionischen grenzflächenaktiven Komponente, welche von der Gruppe ausgewählt ist, die aus anionischen grenzflächenaktiven Mitteln und amphoteren grenzflächenaktiven Mitteln besteht, welche amphoteren grenzflächenaktiven Mittel beim pH-Wert der Zusammensetzung anionisch sind;
   (b) 0,05 Gew.-% bis 10 Gew.-% von einem dispergierten, unlöslichen, nichtflüchtigen, nichtionischen, haarkonditionierenden Mittel aus Silikonbasis;
   (c) 0,05 Gew.-% bis 5 Gew.-% von einem wasserlöslichen, organischen, haarkonditionierenden Mittel auf Basis von kationischem Polymer mit einer kationischen Ladungsdichte von 0,9 mÄqu./g bis 4 mÄqu./g;
   (d) 0,05 Gew.-% bis 5 Gew.-% von einer organischen, nicht flüchtigen, wasserunlöslichen Flüssigkeit, welche von der Gruppe ausgewählt ist, die aus Kohlenwasserstoffölen, Fettestern mit mindestens 10 Kohlenstoffatomen, welche Fettester von der aus Alkyl- und Alkenylestern von Fettsäuren, Alkyl- und Alkenylestern von Fettalkoholen, Dicarbonsäureestern, Tricarbonsäureestern und Mono-, Di- und Triglyceriden bestehenden Gruppe ausgewählt sind, und aus Gemischen hievon besteht; und
   (e) einen wäßrigen Träger umfaßt.

2. Haarkonditionierendes Shampoo nach Anspruch 1, wobei die genannte kationische Ladungsdichte von 1,0 mÄqu./g bis 3,0 mÄqu./g, vorzugsweise von 1,1 mÄqu./g bis 2,0 mÄqu./g, stärker bevorzugt von 1,2 mÄqu./g bis 2,0 mÄqu./g beträgt.

3. Haarkonditionierende Shampoozusammensetzung nach Anspruch 1 oder 2, welche ferner ein Suspendierungsmittel für das genannte haarkonditionierende Mittel auf Silikonbasis umfaßt.

4. Haarkonditionierende Shampoozusammensetzung nach Anspruch 1, 2 oder 3, wobei das genannte kationische Polymer in der genannten Zusammensetzung eine komplexe Koazervatphase darstellt oder bei der Verdünnung des genannten Shampoos mit Wasser ein komplexes Koazervat ausbildet.

5. Shampoozusammensetzung nach Anspruch 4, wobei das genannte kationische Polymer bei Verdünnung des Shampoos mit Wasser in einem Gewichtsverhältnis von Wasser zur Shampoozusammensetzung von 20:1 in einer komplexen Koazervatform vorliegt.

6. Shampoozusammensetzung nach Anspruch 1, 2, 3, 4 oder 5, wobei das genannte haarkonditionierende Mittel auf Silikonbasis Polydimethylsiloxan umfaßt.

7. Shampoozusammensetzung nach Anspruch 1, 2, 3, 4, 5 oder 6, wobei das genannte haarkonditionierende Mittel auf Basis von kationischem Polymer von der Gruppe ausgewählt ist, die aus kationischen Polysacchariden und kationische Vinylmonomere umfassenden Polymeren besteht, vorzugsweise wobei das genannte haarkonditionierende Mittel auf Basis von kationischem Polymer von der aus kationischer Cellulose, kationischer Stärke, kationischem Guar, Copolymeren aus 1-Vinyl-2-pyrrolidon und 1-Vinyl-3-methylimidazoliumsalz, Copolymeren aus Acrylamid und Dimethyldiallylammoniumsalz und Copolymeren aus 1-Vinyl-2-pyrrolidon und Dimethylaminoethylmethacrylat bestehenden Gruppe ausgewählt ist.

8. Shampoozusammensetzung nach Anspruch 1, 2, 3, 4, 5, 6 oder 7, umfassend etwa 10% bis etwa 25% an anionischen grenzflächenaktiven Mitteln, etwa 0,5% bis etwa 5% an einem haarkonditionierenden Mittel auf Silikonbasis, etwa 0,1% bis etwa 4% an haarkonditionierendem Mittel auf Basis von kationischem Polymer und etwa 0,2% bis etwa 3% von einem haarkonditionierenden Mittel auf Basis einer öligen Flüssigkeit.

9. Verfahren zur Shampoonierung von Haar, welches Verfahren das Aufbringen auf das Haar von einer wirksamen Menge der Zusammensetzung nach Anspruch 1, 2, 3, 4, 5, 6, 7 oder 8 zur Reinigung und Konditionierung des Haares und das anschließende Abspülen der genannten Zusammensetzung vom Haar umfaßt.

## Revendications

1. Composition de shampooing conditionneur pour les cheveux, caractérisée en ce qu'elle comprend:

   (a) de 5% à 50%, en poids, d'un constituant tensioactif anionique choisi dans le groupe constitué par les tensioactifs anioniques et les tensioactifs amphotères qui sont anioniques au pH de la composition;
   (b) de 0,05% à 10%, en poids, d'un agent de conditionnement des cheveux de type silicone non ionique, dispersé, insoluble, non volatil;
   (c) de 0,05% à 5%, en poids, d'un agent de conditionnement des cheveux de type polymère cationique, organique, soluble dans l'eau, ayant une densité de charge cationique de 0,9 méq/g à 4 méq/g;
   (d) de 0,05% à 5%, en poids, d'un liquide organique, non volatil, insoluble dans l'eau, choisi dans le groupe constitué par les huiles hydrocarbonées, les esters gras comportant au moins 10 atomes de carbone, choisis dans le groupe constitué par les esters alkyliques et alcényliques d'acides gras, les esters alkyliques et alcényliques d'alcools gras, les esters d'acides dicarboxyliques, les esters d'acides tricarboxyliques, et les mono-, di- et tri-glycérides, et leurs mélanges; et
   (e) un véhicule aqueux.

2. Composition de shampooing conditionneur pour les cheveux selon la revendication 1, dans laquelle ladite densité de charge cationique est de 1,0 méq/g à 3,0 méq/g, de préférence de 1,1 méq/g à 2,0 méq/g, mieux encore de 1,2 méq/g à 2,0 méq/g.

3. Composition de shampooing conditionneur pour les cheveux selon la revendication 1 ou 2, comprenant, en outre, un agent de mise en suspension pour ledit agent de conditionnement des cheveux de type silicone.

4. Composition de shampooing conditionneur pour les cheveux selon la revendication 1, 2 ou 3, dans laquelle ledit polymère cationique est une phase de coacervat complexe dans ladite composition ou bien forme un coacervat complexe par dilution dudit shampooing avec de l'eau.

5. Composition de shampooing selon la revendication 4, dans laquelle ledit polymère cationique existe sous forme de coacervat complexe par dilution du shampooing avec de l'eau dans un rapport pondéral eau/shampooing dans la composition de 20:1.

6. Composition de shampooing selon la revendication 1, 2, 3, 4 ou 5, dans laquelle ledit agent de conditionnement des cheveux de type silicone comprend du polydiméthylsiloxane.

**7.** Composition de shampooing selon la revendication 1, 2, 3, 4, 5 ou 6, dans laquelle ledit agent de conditionnement des cheveux de type polymère cationique est choisi dans le groupe constitué par les polysaccharides cationiques et les polymères comprenant des monomères vinyliques cationiques, de préférence ledit agent de conditionnement des cheveux de type polymère cationique est choisi dans le groupe constitué par la cellulose cationique, l'amidon cationique, le guar cationique, les copolymères de 1-vinyl-2-pyrrolidone et de sel de 1-vinyl-3-méthylimidazolium, les copolymères d'acrylamide et de sel de diméthyldiallylammonium, et les copolymères de 1-vinyl-2-pyrrolidone et de méthacrylate de diméthylaminoéthyle.

**8.** Composition de shampooing selon la revendication 1, 2, 3, 4, 5, 6 ou 7, comprenant d'environ 10% à environ 25% de tensioactifs anioniques, d'environ 0,5% à environ 5% d'agent de conditionnement des cheveux de type silicone, d'environ 0,1% à environ 4% d'agent de conditionnement des cheveux de type polymère cationique et d'environ 0,2% à environ 3% d'agent de conditionnement des cheveux de type liquide huileux.

**9.** Procédé de lavage des cheveux, ledit procédé comprenant l'application, sur les cheveux, d'une quantité efficace de la composition de la revendication 1, 2, 3, 4, 5, 6, 7 ou 8 pour nettoyer et conditionner les cheveux, puis le rinçage des cheveux pour éliminer ladite composition.